# EUROPÄISCHE PATENTSCHRIFT

(11) **EP 0 742 726 B1**
(45) Veröffentlichungstag und Bekanntmachung des Hinweises auf die Patenterteilung: **16.08.2000**
(21) Anmeldenummer: 95905107.9
(22) Anmeldetag: 23.12.1994
(51) Int. Cl.: A61M 5/14, A61M 39/10

(54) **Microkatheter-Set**
Micro-catheter set
Ensemble micro-cathéter

(30) Priorität: 05.02.1994 DE 4403630
(43) Veröffentlichungstag der Anmeldung: 20.11.1996
(73) Patentinhaber: Lang, Volker, Prof. Dr., D-82131 Gauting (DE)
(72) Erfinder: Lang, Volker, Prof. Dr., D-82131 Gauting (DE)
(74) Vertreter: Laufhütte, Dieter, Dr.-Ing.
(86) Internationale Anmeldenummer: EP9404298
(87) Internationale Veröffentlichungsnummer: WO9520987

(56) Entgegenhaltungen:
- EP-A- 0 372 277
- WO-A-91/18632
- FR-A- 2 395 037
- FR-A- 2 452 653
- FR-A- 2 522 969

## Beschreibung

Die Erfindung betrifft ein Microkatheter-Set gemäß dem Oberbegriff des Anspruchs 1.

Aus der WO-A-9118632 ist bereits ein Katheter-Set gemäß dem Oberbegriff des Anspruchs 1 bekannt. Dieses Katheter-Set weist ein großes Totraumvolumen auf. Darüber hinaus beinhaltet es einen aufwendig konstruierten Ventilmechanismus, der im klinischen Betrieb störanfällig ist.

Die moderne Intensivmedizin benötigt nun vor allem im Bereich der Neonatologie bei der Therapie sehr unreifer Frühgeborener, die noch nicht oral mit Milch ernährt werden können, die Möglichkeit der parenteralen Ernährung mit hochprozentigen, nährstoffreichen Infusionslösungen, die nur zentral über großlumige venöse Blutgefäße über Katheter appliziert werden können. Eine Kathetertechnik, die sich vor allem im Bereich der Neonatologie besonders bewährt hat, ist diejenige nach Shaw, die Micro-Epicutaneo-Cava-Katheter verwendet. Entsprechende Katheter sind innerhalb eines Sets im Handel erhältlich. Die verschiedenen Infusionslösungen werden dabei über Verteiler und Adapter über den angeschlossenen Microkatheter meist mit Hilfe von dosiergenauen Spritzenpumpen in eine große zentrale Körnervene infundiert.

Folgende ungelöste klinisch-pharmakologische Probleme ergeben sich aber hierbei: Das Microkatheter-Set bestehend aus Luer-Lock-Adapter, Verbindungsschlauch, Easy-Lock-Katheterverbindung und Microkatheter weisen zusammen ein zu großes Totraumvolumen auf. Das bedeutet aber, vor allem wenn hochwirksame kreislaufaktive Pharmaka (z.B. Katecholamine) der Infusionsflüssigkeit zugesetzt sind - was in der Praxis sehr häufig vorkommt -, daß z.B. beim Durchspülen eines partiell oder vollständig verstopften Katheters oder bei Medikamentenapplikation, dieses große Totraumflüssigkeitsvolumen mit kreislaufwirksamen Pharmaka als Bolus innerhalb kurzer Zeit in die Blutbahn gelangt. Schwere Kreislaufsensationen, bei kleinen Frühgeborenen eventuell gefolgt von lebensbedrohlichen Hirnblutungen, sind mögliche Folgen. Aus diesen genannten Gründen dürfen die Microkatheter bei partieller oder vollständiger Verstopfung nur durchgespült werden, wenn pharmakologisch weitgehend indifferente Infusionslösungen Anwendungen finden. Dasselbe gilt auch für die Medikamentenapplikation. Dies bedeutet aber in praxi, daß bei Patienten ein zweiter venöser Zugang geschaffen werden muß, was insbesondere bei kleinen Früh- und Neugeborenen sehr belastend für den Patienten und den behandelnden Arzt ist. Dies gilt auch für eine andere Alternative, nämlich die Verwendung von doppellumigen zentralen Venenkathetern.

Aus der FR-A-25 22 969 ist ein totraumminimierter Luer-Lock-Konnektor bekannt.

Schließlich ist aus der FR-A-23 95 037 eine totraumminimierte Easy-Lock-Katheterverbindung bekannt.

Aufgabe der Erfindung ist es, ein Microkatheter-Set gemäß dem Oberbegriff des Hauptanspruches derart weiterzubilden, daß die zuvor genannten klinischen Probleme gelöst werden können.

Diese Aufgabe wird mittels eines gattungsgemäßen Microkatheter-Sets gelöst, das die kennzeichnenden Merkmale des Hauptanspruches aufweist.

Weitere vorteilhafte Ausbildungen der erfindungsgemäßen Lösung ergeben sich aus den sich an den Hauptanspruch anschließenden Unteransprüchen.

Weiter Einzelheiten und Vorteile der Erfindung werden an Hand eines in der Zeichnung dargestellten Ausführungsbeispieles näher erläutert. Es zeigen:
- Fig. 1:: eine perspektivische Ansicht des erfindungsgemäßen Microkatheter-Sets,
- Fig. 2:: einen Längsschnitt eines Microkatheters mit eingesetztem Röhrchen und
- Fig. 3:: eine Ausführungsform einer im erfindungsgemäßen Mikrokatheter-Set verwendeten Easy-Lock-Katheterverbindungsvorrichtung.

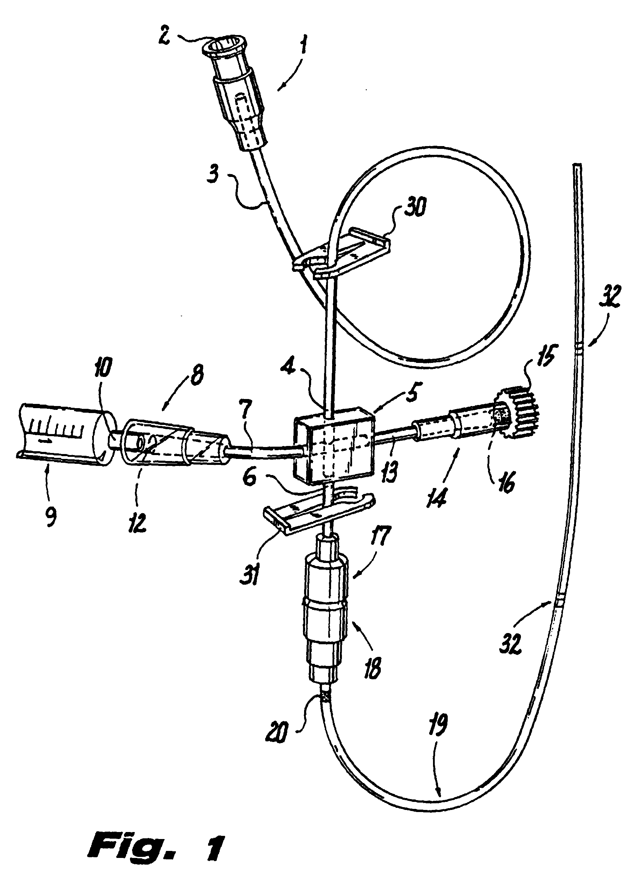

Figur 1 zeigt zusammengebaut, perspektivisch das sterile totraumminimierte Epicutaneo-Cava-Microkatheter-Set zum einmaligen Gebrauch. Es besteht aus einem weiblichen, totraumarm ausgebildeten Norm-Luer-Lock-Adapter 1, der aus durchsichtigem Kunststoff hergestellt ist und dessen zylindrischer, sich konisch verengender Hohlraum 2 zur Aufnahme des Konus eines männlichen Norm-Luer-Lock-Konnektors dient. Dieser Hohlraum 2 ist nur so tief ausgebildet, daß handelsübliche und in ihrer Länge etwas variierende männliche Luer-Lock-Konen eben noch sicher, flüssigkeitsdicht verriegelt werden können. Dadurch läßt sich der zwischen Konusende und Boden des zylindrischen Hohlraums 2 bildende Totraum auf ein Minimum von 25 bis 50 µl reduzieren. Von diesem Boden des zylindrischen Hohlraums führt ein kleinlumiger, dickwandiger, jedoch flexibler und durchsichtiger Kunststoffschlauch 3 (Innendurchmesser: 0,5 mm, Länge: 100 bis 200 mm) zu einem quaderförmigen Vierfachverbindungsstück 5 aus glasklarem Kunststoff. In dem Vierfachverbindungsstück 5 sind Bohrungen vorgesehen, die sich im rechten Winkel kreuzen. An zwei sich gegenüberliegenden Enden ist einmal das Ende 4 des Schlauches 3 und zum anderen ein kurzes, kleinlumiges Verbindungsschlauchstück 6, das zu der aus zwei Teilen 17, 18 zusammengeschraubten Easy-Lock-Verbindung für den Microkatheter 19 eingesetzt. In der waagrecht verlaufenden Bohrung des Vierfachverbinders sind auf der einen Seite ein kleinlumiger, kurzer Verbindungsschlauch 7 mit angefügtem totraumarmen, weiblichen Luer-Lock-Konnektor 8 mit aufgesetzter Einmalspritze 9 und auf der anderen Seite ein entsprechender Verbindungsschlauch 13 mit Konnektor 14 angeschlossen.

Der Fig. 1 ist zu entnehmen, daß die in die totraumarmen weiblichen Luer-Lock-Konnektoren 8 bzw. 14 eingeführten männlichen Luer-Lock-Konen 10 der Einmalspritze 9 bzw. des drehverriegelten Luer-Lock-Verschlußstopfens 15 diese nicht vollständig ausfüllen, sondern einen etwas unterschiedlich großen Totraum 12 bzw. 16, in den die Verbindungsschläuche 7 bzw. 13 einmünden, entstehen lassen.

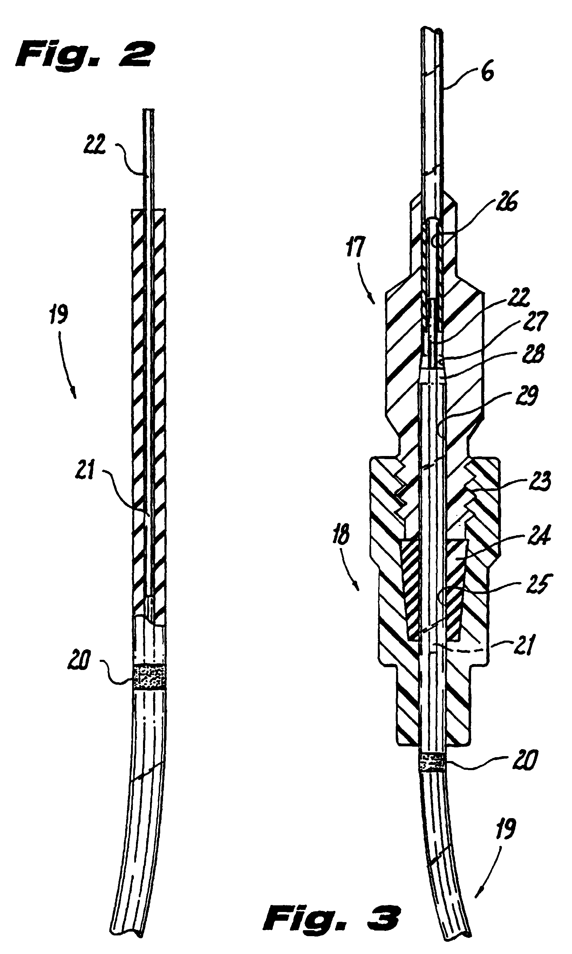

Fig. 2 zeigt im Längsschnitt einen Microkatheter 19 mit einer Ringmarkierung 20 und Längenmarken 32. Der Microkatheter 19 kann aus einem röntgenkontrastgebenden, weichen Silikongummi bzw. Polyurethan gefertigt sein. Der Microkatheter 19 ist auf ein Röhrchen aus Nirostastahl 21 unter Spannung aufgeschoben, wobei bei dem Röhrchen, bezogen auf dessen Gesamtlänge, das vordere Fünftel 22 freigelassen wird. Dieser vordere Anteil dient zum Einschieben in den Easy-Lock-Verbinder 17,18.

Fig. 3 zeigt im Längsschnitt die Easy-Lock-Katheterverbindung im montierten Zustand. In das Teil 17 des Easy-Lock-Verbinders ist der Verbindungsschlauch 6 eingefügt. In seinem engen Lumen 26 sitzt das Vorderteil 22 des Nirostastahlröhrchens 21. Am unteren Ende weist das Teil 17 der Easy-Lock-Verbindung einen Gewindestutzen 23 auf, der zentral einen weiten Einführkanal 29 für den auf das Stahlröhrchen aus Nirosta® aufgeschobenen Microkatheter 19 aufweist, der sich in einem Einführtrichter 27 fortsetzt, der schließlich im Lumen des Verbindungsschlauches 6 endet. Da der Microkatheter 19 beim Zusammenschrauben des Verbinders 17,18 in diesen Einführungstrichter 27 gepreßt wird, bildet er dort einen Dichtwulst 28 aus. Der untere Teil 18 des Easy-Lock-Verbinders stellt eine Plastik-Gewindehülse dar, die sich nach unten zu konisch verjüngt und in diesem Teil einen konischen Gummistopfen 24 mit zentraler Bohrung 25 aufnimmt. In diese zentrale Bohrung 25 ist der Microkatheter mit seinem Teil 21 so weit eingeführt, daß seine Ringmarke 20 eben noch am Rand der unteren Öffnung von Teil 18 sichtbar ist.

Die Funktion des Microkatheter-Sets im Einsatz kann an Hand der Figuren 1 bis 3 wie folgt beschrieben werden. Das sterile Microkatheter-Set mit integrierter Durchspül- und Medikamentenapplikationsvorrichtung wird mit seinem totraumreduzierten, weiblichen Luer-Lock-Konnektor 1 an ein Spritzenpumpeninfusionssystem mit männlichem Luer-Lock-Konnektor (hier nicht dargestellt) angeschlossen. Nach dem Verriegeln von weiblichem und männlichem Konnektor entsteht immer, wie schon beschrieben, ein Resttotraumvolumen, das von der Paßgenauigkeit des männlichen Konnektorkonus abhängt und ca. 25 bis 50 µl beträgt. Durch den angeschlossenen englumigen Verbindungsschlauch 3 (mit einem Innendurchmesser 0,5 mm) werden noch weitere 38 µl, durch den Vierfachverbinder 5, weitere 7 µl, durch den Verbindungsschlauch 6, weitere 4 µl und schließlich durch den totraumminimierten Easy-Lock-Verbinder 17,18 weitere 5 µl hinzugefügt. Insgesamt ergibt sich ein Totraumvolumen von ca. 54 µl. In Abhängigkeit von der Länge und dem Innendurchmesser des Microkatheters ist hier noch ein durch diese bedingtes Totraumvolumen hinzuzurechnen. Bei dem verwendeten Microkatheter müssen beispielsweise bei 150 mm Länge, noch 10,5 µl Volumen hinzugerechnet werden. Beim Freispülen eines z. B. teilverstopften Microkatheters werden dem Patienten innerhalb kurzer Zeit insgesamt ca. 90 bis 120 µl Infusionsflüssigkeit mit eventuell zugesetzten hochwirksamen Pharmaka aus dem Totraumvolumen zugeführt, was zu lebensgefährlichen Überdosierungen führen kann. Bei den heute erhältlichen Microkatheter-Sets, bei denen all diese erwähnten speziellen totraumreduzierenden Maßnahmen nicht vorgenommen sind, muß man sogar vergleichs-weise mit mindestens 240 µl Totraumvolumen rechnen.

Um diese erwähnten großen Totraumvolumina, die klinisch nicht nur ein Durchspülen des Katheter-Set, sondern auch eine Medikamentenapplikation weitgehend verbieten, zu vermeiden, wurde bei dem erfindungsgemäßen Microkatheter-Set, wie es in Fig. 1 dargestellt wurde, patienten-katheternah der Vierfachverbinder 5 zur Durchspülung und Medikamentenapplikation eingefügt. Soll der Microkatheter 19 beispielsweise freigespült werden oder, was auch häufig erwünscht ist zur Medikamentenapplikation benutzt werden, dann kann dies jetzt gefahrlos für den Patienten auf folgende Weise vorgenommen werden (vergleiche Fig. 1).

Vom weiblichen Luer-Lock-Verbinder 8 wird der Luer-Lock-Verschlußstopfen (nicht dargestellt) abgenommen und dafür eine, z.B. 2 ml Einmalspritze 9 gefüllt mit physiologischer Kochsalzlösung aufgesetzt. Jetzt werden zwei Schiebe-Schlauchklemmen 30, 31, die an den Schläuchen 3 und 6 vorgesehen sind, geschlossen und der drehverriegelte Luer-Lock-Verschlußstopfen 15 gelockert und damit der Luer-Konnektor 14 geöffnet. Nun wird mit der Spritze 9 mit physiologischer Kochsalzlösung zuerst der mit Infusionsflüssigkeit gefüllte Totraum 12, das heißt ca. 25 bis 50 µl, dann der Verbindungsschlauch 7, das heißt ca. 7 µl, ein Stück der waagrechten Bohrung in Verteiler 5, das heißt ca. 7µl, der Verbindungsschlauch 13, das heißt ca. 7 µl und der Totraum 16 des Luer-Konnektors 14, das heißt 25 bis 50 µl, insgesamt also schließlich ca. 75 bis 175 µl Totraumvolumen-Flüssigkeit ausgespült.

Wird nun der Luer-Lock-Verschlußstopfen wieder verriegelt und die Schiebe-Schlauchklemme 31 geöffnet, dann kann über den Luer-Konnektor 8, am vorteilhaftesten mit Hilfe einer jetzt dort angeschlossenen Infusionsspritzenpumpe, die Durchspülung oder die gewünschte Medikamentenapplikation langsam und kontinuierlich in einer oder mehreren Minuten über den Microkatheter 19 vorgenommen werden. Das mit Infusionslösung gefüllte gesamte Totraumvolumen von jetzt 19 µl ergibt sich dabei aus dem Totraumvolumen des verwendeten 150 mm langen Microkatheters mit 10,5 µl, dem des Easy-Lock-Verbinders mit 5 µl und dem des Verbindungsschlauches 6 mit 3,5 µl (vergleiche Fig. 1).

Wird z. B. an der am Konnektor 8 angeschlossenen Infusionsspritzenpumpe eine Infusionsgeschwindigkeit von 1 ml pro Stunde für die ersten ein bis zwei Minuten nach Infusionsbeginn eingestellt, dann können in praxi selbst bei allerkleinsten Frühgeborenen Katheter-Medikamentenapplikationen und -durchspülungen gefahrlos vorgenommen werden. Hier werden nur noch 19 µl Infusionslösung und die auch noch protrahiert innerhalb dieser Zeitspanne in das Venensystem des Patienten abgegeben.

Soll nach Medikamentengabe oder nach Durchspülen die allgemeine Infusionstherapie fortgesetzt werden, dann kann die Medikamenten-Infusionspumpe vom Luer-Lock-Konnektor 8 dekonnektiert und dieser mit einem Luer-Lock-Verschlußstopfen, Stopfen 15 entsprechend, verschlossen werden. Nachfolgend wird die Schiebe-Schlauchklemme 30 geöffnet und damit der Infusionsfluß über den Luer-Lock-Adapter 1 und den Verbindungsschlauch 3 wieder freigegeben.

## Patentansprüche

1. Microkatheter-Set, insbesondere Epicutaneo-Cava-Microkatheter-Set, bestehend aus einem Katheter (19), einer Easy-Lock-Katheter-Verbindungsvorrichtung (17, 18) mit einem Schlauch (6) und einem Norm-Luer-Lock-Konnektor (1), wobei dicht vor der Easy-Lock-Katheter-Verbindungsvorrichtung (17, 18) patientenkatheternah eine Durchspül-Medikamentenapplikations-Vorrichtung (9) vorgesehen ist, mittels derer der Katheter (19) durchspülbar ist und Medikamente applizierbar sind,
**dadurch gekennzeichnet,**
daß zur generellen Totraumminimierung des Microkatheter-Sets totraumminimierte Norm-Luer-Lock-Konnektoren (1, 8, 14), Microlumenschläuche (3, 6, 7, 13) und eine totraumminimierte Easy-Lock-Katheter-Verbindungsvorrichtung (17, 18) eingesetzt werden und der Katheter (19) als Microkatheter (19) ausgeführt ist,
wobei die Easy-Lock-Katheter-Verbindungsvorrichtung (17, 18) ein langes Nirosta®-Stahlröhrchen (21) umfaßt, auf das der elastische Microkatheter (19) klemmend soweit aufgeschoben ist, daß der vordere Teil (22) des Nirostastahlröhrchens (21) unbedeckt ist und
des weiteren aus zwei miteinander verschraubbaren Teilen (17,18) derart aufgebaut ist, daß die Spitze des vorderen Teils (22) des Nirosta®-Stahlröhrchens (21) nach dem Zusammenschrauben der beiden Teile (17, 18) in das enge Lumen (26) des an dem einen verschraubbaren Teil (17) angeschlossenen Microlumenschlauchs (6) dichtend eingepreßt ist.

2. Microkatheter-Set nach Anspruch 1, dadurch gekennzeichnet,
daß ein Teil (17) der zweigeteilten Easy-Lock-Katheter-Verbindungsvorrichtung (17, 18) einen Einführkanal (29) zur Aufnahme des auf das Nirosta®-Stahlröhrchen (21) aufgeschobenen Microkatheters (19) sowie einen sich daran anschließenden Einführtricher (27) aufweist, der nach Einführung des unbedeckten Teils (22) des Nirosta®-Stahlröhrchens (21) den aufgepreßten elastischen Microkatheter (19) zu einem Dichtwulst (28) umformt.

3. Microkatheter-Set nach Anspruch 1 oder 2, dadurch gekennzeichnet, daß die Durchspül-Medikamentenapplikations-Vorrichtung (9) über einen Vierfachverteiler (5) oder einen Vierwegehahn mit dem Microlumenschlauch (6) verbunden ist.

4. Microkatheter-Set nach Anspruch 3, dadurch gekennzeichnet, daß der Vierfachverteiler (5) aus Kunststoff gefertigt ist, eine vorzugsweise quaderförmige oder scheibenförmige Gestalt aufweist und von zwei in einem rechten Winkel sich kreuzenden Kanälen durchzogen ist.

5. Microkatheter-Set nach Anspruch 3 oder 4, dadurch gekennzeichnet, daß an dem senkrecht verlaufenden Kanal jeweils ein zuführender (3) und ein abführender Microlumenschlauch (6) dicht schließend eingefügt ist.

6. Microkatheter-Set nach Anspruch 5, dadurch gekennzeichnet, daß dem senkrecht verlaufenden zuführenden Kanal vorgeschaltet zusätzlich ein Einwegventil mit oder ohne Vorspannung mit Flußrichtung zum Patienten angeordnet ist.

7. Microkatheter-Set nach einem der Ansprüche 3 bis 6, dadurch gekennzeichnet, daß an einen waagrecht verlaufenden Kanal des Vierfachverteilers (5) bzw. Vierwegehahns an beiden Enden kurze Microlumenverbindungsschläuche (7, 13) dicht schließend eingesetzt sind und daß diese an ihrem freien Ende je einen weiblichen totraumminimierten Norm-Luer-Lock-Konnektor (8, 14) tragen, der durch einen drehverriegelten Luer-Lock-Verschlußstopfen (15) verschließbar ist.

8. Microkatheter-Set nach einem der Ansprüche 1 bis 7, dadurch gekennzeichnet, daß der totraumminimierter Norm-Luer-Lock-Konnektor derart ausgebildet ist, daß beim weiblichen Norm-Luer-Lock-Konnektor (1, 8, 14) durch eine am Boden eines zylindrischen sich konisch verengenden Hohlraums (2) eine elastische Scheibe mit zentraler Ausnehmung derart vorgesehen ist, daß der immer bei Konnektion durch Fertigungstoleranzen der männlichen und weiblichen Konnektoren sonst auftretende Totraum durch diese elastische Scheibe vollständig ausgefüllt ist.

9. Microkatheter-Set nach einem der Ansprüche 1 bis 7, dadurch gekennzeichnet, daß der totraumminimierte Norm-Luer-Lock-Konnektor derart ausgebildet ist, daß beim männlichen Konnektor (10) durch eine auf dessen Konus an der Stirnseite aufgebrachte elastische Scheibe mit zentraler Ausnehmung der Totraum bei der Konnektion vollständig durch diese Scheibe ausgefüllt ist.

10. Microkatheter-Set nach Anspruch 7, dadurch gekennzeichnet, daß der Luer-Lock-Verschlußstopfen (15) ein verlängertes Gewinde zur Drehverriegelung aufweist, so daß eine oder zwei Drehungen ausgeführt werden können, bis es zur Verriegelung und zum dichten Verschluß des Konnektors kommt.

## Claims

1. Micro-catheter set, in particular epicutaneo-cava micro-catheter set, comprising a catheter (19), an easy-lock catheter connection device (17, 18) with a tube (6) and a standard Luer lock connector (1), with a flushing and medicament application device (9) being provided close in front of the easy-lock catheter connection device (17, 18) near the catheter of the patient, by means of which device (9) the catheter (19) can be flushed and medicaments can be applied, characterized in that for generally minimizing the dead space of the micro-catheter set, dead-space-minimized standard Luer lock connectors (1, 8, 14), micro-lumen tubes (3, 6, 7, 13) and a dead-space-minimized easy-lock catheter connection device (17, 18) are used and the catheter (19) is designed as a micro-catheter (19), the easy-lock catheter connection device (17, 18) including a long Nirosta® steel tubing (21) onto which the elastic micro-catheter (19) can be tightly pushed until the front part (22) of the Nirosta steel tubing (21) is uncovered, and moreover being made up of two parts (17, 18), which can be screwed together, in such a way that the tip of the front part (22) of the Nirosta® steel tubing (21), after the two parts (17, 18) have been screwed together, is pressed tightly into the narrow lumen (26) of the micro-lumen tube (6) attached to the one screw-on part (17).

2. Micro-catheter set according to Claim 1, characterized in that a part (17) of the two-part easy-lock catheter connection device (17, 18) has an insertion channel (29) for receiving the micro-catheter (19) pushed onto the Nirosta® steel tubing (21) and an adjoining insert funnel (27) which, after insertion of the uncovered part (22) of the Nirosta® steel tubing (21), deforms the pressed-on elastic micro-catheter (19) to a sealing bead (28).

3. Micro-catheter set according to Claim 1 or 2, characterized in that the flushing and medicament application device (9) is connected to the micro-lumen tube (6) by a four-way distributor (5) or a four-way valve.

4. Micro-catheter set according to Claim 3, characterized in that the four-way distributor (5) is made of plastic, has a preferably square or disc-shaped configuration and has two channels which intersect at right angles passing through it.

5. Micro-catheter set according to Claim 3 or 4, characterized in that an intake micro-lumen tube (3) and an outlet micro-lumen tube (6) respectively are fitted sealingly on the vertically extending channel.

6. Micro-catheter set according to Claim 5, characterized in that a one-way valve is also arranged upstream of the vertically extending intake channel with or without prestressing, with direction of flow to the patient.

7. Micro-catheter set according to one of Claims 3 to 6, characterized in that short micro-lumen connection tubes (7, 13) are fitted sealingly on a horizontally extending channel of the four-way distributor (5) or four-way valve at both ends, and in that these each bear at their free end a female, dead-space-minimized standard Luer lock connector (8, 14) which can be closed by a rotationally locked Luer lock closure stopper (15).

8. Micro-catheter set according to one of Claims 1 to 7, characterized in that the dead-space-minimized standard Luer lock connector is designed in such a way that an elastic disc with central opening is provided at the bottom of a cylindrical, conically tapering cavity (2) in the female standard Luer lock connector (1, 8, 14) such that the dead space which otherwise always occurs upon connection as a result of manufacturing tolerances of the male and female connectors is filled completely by this elastic disc.

9. Micro-catheter set according to one of Claims 1 to 7, characterized in that the dead-space-minimized standard Luer lock connector is designed in such a way that in the male connector (10), as a result of an elastic disc with central opening arranged on its cone, on the end side, the dead space upon connection is completely filled by this disc.

10. Micro-catheter set according to Claim 7, characterized in that the Luer lock closure stopper (15) has an extended thread for rotational locking, so that one or two turns can be executed until the connector is locked and tightly sealed.

## Revendications

1. Ensemble de micro-cathéter, notamment ensemble de Epicutaneo-Cava micro-cathéter, constitué d'un cathéter (19), d'un dispositif de raccordement de cathéter à verrouillage facile (17, 18) avec un tuyau (6) et un connecteur de verrouillage Luer standard (1), où est prévu peu avant le dispositif de raccordement de cathéter à verrouillage facile (17, 18), à proximité du cathéter côté patient, un dispositif de rinçage et d'administration de médicament (9) au moyen duquel le cathéter (19) peut être rincé et des médicaments peuvent être administrés, caractérisé on ce que, pour réduire généralement à un minimum l'espace mort de l'ensemble de micro-cathéter, sont utilisés des connecteurs de verrouillage Luer standard (1, 8, 14) dont l'espace mort est réduit à un minimum, des tuyaux à micro-lumière (3, 6, 7, 13) et un dispositif de raccordement de cathéter à verrouillage facile (17, 18) dont l'espace mort est réduit à un minimum et où le cathéter (19) est réalisé comme micro-cathéter (19), où le dispositif de raccordement de cathéter à verrouillage facile (17, 18) comprend un tube long en acier Nirosta® (21) sur lequel est poussé et serré le micro-cathéter élastique (19) de telle manière que la partie avant (22) du tube en acier Nirosta® (21) n'est pas recouverte et est constitué en outre de deux parties (17, 18) pouvant être assemblées par vissage de façon que la pointe de la partie avant (22) du tube en acier Nirosta® (21), après le vissage des deux parties (17, 18) est poussé d'une manière étanche dans la lumière étroite (26) du tuyau à micro-lumière (6) raccordé à une partie à visser (17).

2. Ensemble de micro-cathéter selon la revendication 1, caractérisé an ce qu'une partie (17) du dispositif de raccordement de cathéter à verrouillage facile (17, 18) séparé an deux parties présente un canal d'insertion (29) pour recevoir le micro-cathéter (19) poussé sur le tube an acier Nirosta® (21) ainsi qu'une trémie d'insertion (27) faisant suite à celui-ci qui, après l'insertion de la partie non recouverte (22) du tube en acier Nirosta® (21) transforme le micro-cathéter élastique appliqué par pression (19) an un bourrelet d'étanchéité (28).

3. Ensemble de micro-cathéter selon la revendication 1 ou 2, caractérisé en ce que le dispositif de rinçage et d'administration de médicaments (9) est relié par un distributeur quadruple (5) ou un robinet à quatre voies au tuyau à micro-lumière (6).

4. Ensemble de micro-cathéter selon la revendication 3, caractérisé on ce que le distributeur quadruple (5) est réalisé an matière synthétique, qu'il a une configuration de préférence en forme de parallélépipède ou de disque et est traversé par deux canaux se croisant à angle droit.

5. Ensemble de micro-cathéter selon la revendication 3 ou 4, caractérisé en ce qu'il est inséré, on fermant d'une manière étanche, dans le canal s'étendant verticalement, respectivement un tuyau à micro-lumière d'amenée (3) et d'évacuation (6).

6. Ensemble de micro-cathéter selon la revendication 5, caractérisé en ce qu'il est disposé on amont du canal d'amenée s'étendant verticalement de plus une vanne à une voie avec ou sans précontrainte, à direction d'écoulement vers le patient.

7. Ensemble de micro-cathéter selon l'une des revendications 3 à 6, caractérisé en ce que sont insérés, an fermant d'une manière étanche, dans un canal s'étendant horizontalement du distributeur quadruple (5) respectivement du robinet à quatre voies, aux deux extrémités, de courts tuyaux de raccordement à micro-lumière et que ceux-ci portent à leur extrémité libre chacun un connecteur de verrouillage Luer standard femelle (8, 14) dont l'espace mort est réduit à un minimum et qui peut être fermé par un bouchon de fermeture Luer (15) verrouillé en rotation.

8. Ensemble de micro-cathéter selon l'une des revendications 1 à 7, caractérisé on ce que le connecteur de verrouillage Luer standard dont l'espace mort est réduit à un minimum est réalisé de telle sorte qu'il est prévu dans le connecteur de verrouillage Luer standard femelle (1, 8, 14), au fond d'un espace creux cylindrique (2) à rétrécissement conique un disque élastique à évidement central, de telle sorte que l'espace mort sinon toujours produit, du fait des tolérances de fabrication lors de la connexion, des connecteurs mâle et femelle soit entièrement rempli par ce disque élastique.

9. Ensemble de micro-cathéter selon l'une des revendications 1 à 7; caractérisé en ce que le connecteur de verrouillage Luer standard à espace mort minimisé est réalisé de façon que dans le connecteur mâle (10), par un disque élastique à évidement central appliqué sur le cône de celui-ci au côté frontal, l'espace mort lors de la connexion soit entièrement rempli par ce disque.

10. Ensemble de micro-cathéter selon la revendication 7, caractérisé en ce que le bouchon de verrouillage Luer (15) présente un filetage allongé pour le verrouillage par rotation, de telle sorte qu'une ou deux rotations peuvent être faites jusqu'à ce qu'on obtienne le verrouillage et la fermeture étanche du connecteur.
